# EUROPEAN PATENT SPECIFICATION

(11) **EP 3 204 775 B1**
(45) Date of publication and mention of the grant of the patent: **29.11.2023**
(21) Application number: 15778680.7
(22) Date of filing: 12.10.2015
(51) Int. Cl.: G01N 33/574, G01N 33/74, G01N 27/48, G01N 27/00

(54) **ASSAY FOR FGF2 SECRETION AND SIGNALING**
TEST AUF FGF2-SEKRETION UND -SIGNALISIERUNG
DOSAGE POUR LA SÉCRÉTION ET LA SIGNALISATION DE FGF2

(30) Priority: 10.10.2014 GB 201417961
(43) Date of publication of application: 16.08.2017
(73) Proprietor: Universität Heidelberg, 69117 Heidelberg (DE)
(72) Inventor: NICKEL, Walter, 69198 Schriesheim (DE)
(74) Representative: Tegethoff, Sebastian
(86) International application number: PCT/EP2015/073591
(87) International publication number: WO 2016/055664

(56) References cited:
- EP-A1- 2 535 410
- WO-A1-2009/096902
- WO-A1-2009/111087
- WO-A1-2010/144696
- JP-A- 2011 126 850
- JP-A- 2011 184 392
- US-A1- 2004 209 246
- LING ZHANG ET AL: "Original Article Propranolol inhibits angiogenesis via down-regulating the expression of vascular endothelial growth factor in hemangioma derived stem cell", INT J CLIN EXP PATHOL, vol. 7, no. 1, 15 December 2013 (2013-12-15), pages 48-55, XP055226042,
- FRANCESCA RIUZZI ET AL: "S100B Engages RAGE or bFGF/FGFR1 in Myoblasts Depending on Its Own Concentration and Myoblast Density. Implications for Muscle Regeneration", PLOS ONE, vol. 7, no. 1, 20 January 2012 (2012-01-20), page e28700, XP055235082, DOI: 10.1371/journal.pone.0028700
- E PESTEREVA ET AL: "PPAR[gamma] agonists regulate the expression of stemness and differentiation genes in brain tumour stem cells", BRITISH JOURNAL OF CANCER, vol. 106, no. 10, 24 April 2012 (2012-04-24), pages 1702-1712, XP055235045, GB ISSN: 0007-0920, DOI: 10.1038/bjc.2012.161
- SANDRA G. VELLEMAN ET AL: "Effects of glypican-1 on turkey skeletal muscle cell proliferation, differentiation and fibroblast growth factor 2 responsiveness", DEVELOPMENT GROWTH AND DIFFERENTIATION., vol. 48, no. 4, 1 May 2006 (2006-05-01), pages 271-276, XP055235168, US ISSN: 0012-1592, DOI: 10.1111/j.1440-169X.2006.00860.x
- André Afl Van Puijenbroek ET AL: "Cell scattering of SK-N-MC neuroepithelioma cells in response to Ret and FGF receptor tyrosine kinase activation is correlated with sustained ERK2 activation", ONCOGENE, vol. 14, no. 10, 1 March 1997 (1997-03-01) , pages 1147-1157, XP055490283, London ISSN: 0950-9232, DOI: 10.1038/sj.onc.1200911

## Description

### FIELD OF THE INVENTION

The present invention relates to a method for the identification of FGF2 secretion and/or signalling and use of the method.

### BACKGROUND OF THE INVENTION

Growth factors belong to a group of proteins that stimulate the growth of specific tissues. Fibroblast growth factors (FGFs) are small polypeptide growth factors and belong to the family of growth factors. They share certain structural characteristics and most of them bind to heparin. Many FGFs contain signal peptides for secretion and are secreted into the extracellular matrix (EM). FGFs bind specific receptors and induce by their binding the activation of intracellular signal cascades.

Epithelial and tumor cells for instance produce FGF2 or basic FGF, which does not contain a signal sequence for secretion. A nuclear localization sequence has been reported upstream of the AUG start codon (Bugler et al., 1991, MCB 11, 4770 - 4777), although the role of nuclear localization remains unclear.

Ramasamy and colleagues (Blood, 2010, Vol. 116, No. 21, p. 432; British Journal of Hematology, 2012, 157, p. 564) describe an in-vitro experimental platform for high throughput analysis of the effect on multiple myeloma cells and the tumor microenvironment in a co-culture setting. They generated eGFP expressing multiple myeloma (MM) cells and other cancer derived cell lines expressing eGFP. The use of eGFP-MM cells allows flow cytometry analysis of cell cycle profile and apoptosis with no significant cellular contamination from co-cultured cells such as fibroblasts, osteoclasts or stromal cells derived from bone marrow aspirates of MM patients. Using cancer derived cell lines, the platform of Ramasamy and colleagues is not appropriate for detecting FGF2.

Flaberg an colleagues (Int. Journal of Cancer, 2011, Vol. 128, No. 21, p. 2793) teach a highthrouput system employing a lymph node metastasis derived cell line that is stably transfected with a constitutive CMV promotor driven eGFP. Other cancer derived cell lines were transfected with recombinant histone H2A-red fluorescent protein. However, the disclosed system is based on cancer cell line and for that reason not suitable for detecting FGF2 secretion.

Fuijata et al (Cancer Science, 2009, Vol. 100, No. 12, p. 2390) disclose a simplified direct co-culture system that is able to quantify populations of cancer cells in co-culture. They established three eGFP expressing pancreatic cancer cell lines and were able to quantify them reliable and reproducible whenever co-cultured with activated pancreatic stellate cells.

The publication by Zhang and colleagues (Zhang et al, Int J Clin Exp Pathol. 2013 Dec 15;7(1):48-55) relates to oral propranolol which been shown to be highly effective for infantile hemangiomas (IHs), and is recommended as the first-line treatment of complicated IHs. However, the therapeutic mechanism(s) still remain unclear. The study shows results for testing hemangioma-derived stem cells for expression of vascular endothelial growth factor (VEGF) in vitro and the inhibition of VEGF expression. The study demonstrates that the application of oral propranolol at a "normal" concentration equivalent to plasma concentration did not inhibit proliferation or promote apoptosis of hemangioma derived stem cells (HemSCs) isolated from IH patients. Oral propranolol suppressed expression of vascular endothelial growth factor (VEGF) and basic Fibroblast Growth Factor (bFGF) in HemSCs in vitro. Morphological, histological and immunohistological improvement were observed in vivo using murine IH model in which HemSCs pre-treated with oral propranolol were implanted into BALB/c-nu mice. In the pre-treated HemSC grafts, mean micro-vessel density (MVD) significantly decreased and protein levels of VEGF markedly decreased, while bFGF was still detectable. The results of the study suggest that oral propranolol inhibited angiogenesis via down-regulating the expression of vascular endothelial growth factor in hemangioma derived stem cell. These findings provide critical insight into the potential mechanisms of oral propranolol action on IH.

Riuzii et al (PLoS One. 2012;7(1):e28700. doi: 10.1371/journal.pone.0028700) show that in high-density myoblast cultures S100B enhances basic fibroblast growth factor (bFGF) receptor 1 (FGFR1) signaling via binding to bFGF and blocks its canonical receptor, receptor for advanced glycation end-products (RAGE), thereby stimulating proliferation and inhibiting differentiation. The publication provides evidence that upon skeletal muscle injury S100B is released from myofibers with maximum release at day 1 post-injury in coincidence with satellite cell activation and the beginning of the myoblast proliferation phase, and declining release thereafter in coincidence with reduced myoblast proliferation and enhanced differentiation. By contrast, levels of released bFGF are remarkably low at day 1 post-injury, peak around day 5 and decline thereafter. The authors also show that in low-density myoblast cultures S100B binds RAGE, but not bFGF/FGFR1 thereby simultaneously stimulating proliferation via ERK1/2 and activating the myogenic program via p38 MAPK. Clearance of S100B after a 24-h treatment of low-density myoblasts results in enhanced myotube formation compared with controls as a result of increased cell numbers and activated myogenic program, whereas chronic treatment with S100B results in stimulation of proliferation and inhibition of differentiation due to a switch of the initial low-density culture to a high-density culture. However, at relatively high doses, S100B stimulates the mitogenic bFGF/FGFR1 signaling in low-density myoblasts, provided bFGF is present. It is proposed that S 100B is a danger signal released from injured muscles that participates in skeletal muscle regeneration by activating the promyogenic RAGE or the mitogenic bFGF/FGFR1 depending on its own concentration, the absence or presence of bFGF, and myoblast density.

Published European Patent Application No. EP 2 535 410 A1 discloses an aptamer having an inhibitory activity on FGF2; a complex containing an aptamer having a binding activity or an inhibitory activity on FGF2, and a functional substance (e.g., affinity substance, labeling substance, enzyme, drug delivery vehicle, or drug and the like); a medicament, diagnostic reagent or label containing an aptamer having a binding activity or an inhibitory activity on FGF2, or a complex containing said aptamer and a functional substance; and the like.

The publication of Pestereva et al. (Br J Cancer. 2012 May 8;106(10):1702-12. doi: 10.1038/bjc.2012.161)relates to brain tumour stem cells (BTSCs) which are a small population of cancer cells that exhibit self-renewal, multi-drug resistance, and recurrence properties. The authors have shown earlier that peroxisome proliferator-activated receptor gamma (PPARγ) agonists inhibit the expansion of BTSCs in T98G and U87MG glioma. In this study, it is analysed the influence of PPARγ agonists on the expression of sternness and differentiation genes in BTSC, which were isolated from T98G and DB29 glioma cells, and cultured in neurobasal medium with epidermal growth factor+basic fibroblast growth factor. Proliferation was measured by WST-1 (4-[3-(4-iodophenyl)-2-(4-nitrophenyl)-2 H-5-tetrazolio]-1,3-benzene disulphonate) and 3H thymidine uptake assays, and gene expression was analysed by quantitative reverse--transcription PCR and Taqman array. The expression of CD133, SRY box 2, and nanog homeobox (Nanog) was also evaluated by western blotting, immunostaining, and flow cytometry. The publication discloses that PPARγ agonists, ciglitazone and 15-deoxy-Δ(12,14)-ProstaglandinJ(2), inhibited cell viability and proliferation of T98G- and DB29-BTSCs. The PPARγ agonists reduced the expansion of CD133(+) BTSCs and altered the expression of sternness and differentiation genes. They also inhibited Sox2 while enhancing Nanog expression in BTSCs. These findings highlight that PPARγ agonists inhibit BTSC proliferation in association with altered expression of Sox2, Nanog, and other sternness genes. Therefore, targeting sternness genes in BTSCs could be a novel strategy in the treatment of glioblastoma.

Published International Patent Application No. WO 2009/096902 A1 teaches an improved efficient method for differentiating stem cells into pancreatic endoderm cells and pancreatic hormone expressing and secreting cells which express Pdx-1 and C-peptide. The application further provides screening methods for detecting factors of interest that will affect the differentiation of the stem cells into pancreatic endoderm cells.

Published International Patent Application No. WO 2009/111087 A1 relates to de-differentiation protocols for generating progenitor cells from adult connective tissue, in particular adult human fibroblasts. The de- differentiation protocols described in this application comprise culturing the differentiated cells with an amount of FGF2 to de-differentiate the cells. These de-differentiated cells may then be cultured and used for experimentation, amplification and clinical applications. The clinical applications include the use of the cells for tissue and cell based therapies.

Published Japanese Patent Application No. JP 2011 184392 A disloses a high-performance and safe melanin production inhibitor, endothelin-1mRNA expression inhibitor, stem cell proliferation factor (SCF) mRNA expression inhibitor, basic fibroblast growth factor (bFGF) mRNA expression inhibitor, glutathione production promotor, damage inhibitor to hydrogen peroxide, and filaggrin production promotor include an extract of a Yakushima hydrangea.

Published Japanese Patent Application No. JP 2011 126850 A discloses a highly safe stem cell growth factor (SCF) mRNA expression increase inhibitor and a basic fibroblast growth factor (bFGF) mRNA expression increase inhibitor which contain a natural extract and are highly safe. An extract from purple rice bran is contained in a stem cell growth factor (SCF) mRNA expression increase inhibitor and a basic fibroblast growth factor (bFGF) mRNA expression increase inhibitor.

Published International Patent Application No. WO 2010/144696 A1 teaches a method comprising incubating pluripotent stem cells in the presence of an amount of one or more Neural Development Factors (NDF) sufficient to generate cells expressing Pax-6, Otx2, Nestin, or a combination, thereby producing neural cells.

The study of Velleman et al. (Dev Growth Differ. 2006 May;48(4):271-6) teaches that heparan sulfate proteoglycan, glypican-1, is a low affinity receptor for fibroblast growth factor 2 (FGF2). Fibroblast growth factor 2 is a potent stimulator of skeletal muscle cell proliferation and an inhibitor of differentiation. Heparan sulfate proteoglycans like glypican-1 are required for FGF2 to transduce an intracellular signal. Understanding the role of glypican-1 in the regulation of FGF2-mediated signaling is important in furthering the understanding of the biological processes involved in muscle development and growth. In said study, a turkey glypican-1 expression vector construct was transfected into turkey myogenic satellite cells resulting in the overexpression of glypican-1. The proliferation, differentiation, and responsiveness to FGF2 were measured in control and transfected cell cultures. The overexpression of glypican-1 in turkey myogenic satellite cells increased both satellite cell proliferation and FGF2 responsiveness, but decreased the rate of differentiation. The current data support glypican-1 modulation of both proliferation and differentiation through an FGF2-mediated pathway.

Published U.S. Patent Application No. US 2004/209246 A1 teaches methods for evaluating antiviral activity and cytotoxicity of a compound comprising providing a target cell population containing a first reporter gene; introducing a second reporter gene into the cell population by integrating the reporter into a replicon of a positive strand RNA virus and making a dual reporter replicon cell line; adding a test compound; incubating the cell population; measuring the responses of the first and second reporter genes; and comparing the responses of the first and second reporter genes in cell populations treated with compound to the responses of the first and second reporter genes in cell populations in the absence of the compound.

The publicaktion by Kottakis and colleagues (Mol Cell. 2011 Jul 22;43(2):285-98) relates to the histone H3K27 methyltransferase EZH2 which plays an important role in oncogenesis, by mechanisms that are incompletely understood. The autohrs show that the JmjC domain histone H3 demethylase NDY1 synergizes with EZH2 to silence the EZH2 inhibitor miR-101. NDY1 and EZH2 repress miR-101 by binding its promoter in concert, via a process triggered by upregulation of NDY1. Whereas EZH2 binding depends on NDY1, the latter binds independently of EZH2. However, both are required to repress transcription. NDY1 and EZH2 acting in concert upregulate EZH2 and stabilize the repression of miR-101 and its outcome. NDY1 is induced by FGF-2 via CREB phosphorylation and activation, downstream of DYRK1A, and mediates the FGF-2 and EZH2 effects on cell proliferation, migration, and angiogenesis. The FGF-2-NDY1/EZH2-miR-101-EZH2 axis described here was found to be active in bladder cancer. These data delineate an oncogenic pathway that functionally links FGF-2 with EZH2 via NDY1 and miR-101.

Fedorov et al (Mol Cell Biol. 1998 Oct;18(10):5780-7) have investigated the role of heterotrimeric G proteins in mediating fibroblast growth factor (FGF)-dependent signals that regulate myogenic differentiation. Pertussis toxin, which ADP-ribosylates and inactivates susceptible G proteins, promotes terminal differentiation in the presence of FGF-2, suggesting that Galpha or Gbeta gamma subunits or both are involved in transducing the FGF-dependent signal(s) that inhibits myogenesis. The autors found that Gbetagamma subunits are likely to be involved since the expression of the C terminus of beta-adrenergic receptor kinase 1, a Gbetagamma subunit-sequestering agent, promotes differentiation in the presence of FGF-2, and expression of the free Gbeta gamma dimer can replace FGF-2, rescuing cells from pertussis toxin-induced differentiation. Addition of pertussis toxin also blocked FGF-2-mediated activation of mitogen-activated protein kinases (MAPKs). Ectopic expression of dominant active mutants in the Ras/MAPK pathway rescued cells from pertussis toxin-induced terminal differentiation, suggesting that the Gbeta gamma subunits act upstream of the Ras/MAPK pathway. It is unlikely that the pertussis toxin-sensitive pathway is activated by other, as yet unidentified FGF receptors since PDGF (platelet-derived growth factor)-stimulated MM14 cells expressing a chimeric receptor containing the FGF receptor-1 intracellular domain and the PDGF receptor extracellular domain were sensitive to pertussis toxin. Our data suggest that FGF-mediated signals involved in repression of myogenic differentiation are transduced by a pertussis toxin-sensitive G-protein-coupled mechanism. This signaling pathway requires the action of Gbeta gamma subunits and activation of MAPKs to repress skeletal muscle differentiation.

The publication of van Pujienbroeck (Oncogene. 1997 Mar 13;14(10): 1147-57) relates to the c-ret proto-oncogene encodes a receptor tyrosine kinase which plays an important role in kidney and enteric nervous system development. Germline mutations in c-ret are responsible for the dominantly inherited cancer syndromes, multiple endocrine neoplasia types 2A and 2B and familial medullary thyroid carcinoma as well as the developmental disorder Hirschsprung's disease. Using SK-N-MC neuroepithelioma cells stably transfected with an EGFR/Ret chimeric receptor, the authors have studied cellular consequences and signalling events following activation of exogenous EGFR/Ret and endogenous FGF and PDGF receptor tyrosine kinases in cells of neuroectodermal origin. The publication shows that Ret activation led to cell scattering, growth inhibition and loss of anchorage-independent growth. Basic FGF, but not PDGF, evoked similar responses in those cells. Nevertheless, activation of all three receptor tyrosine kinases led to ERK2 activation. Analysis of the kinetics of ERK2 activation and downstream events revealed that Ret and FGF receptor activation led to sustained ERK2 activation and SRE transactivation, while PDGF treatment led to transient ERK2 activation and failed to induce SRE transactivation. The results suggest that sustained, but not transient ERK2 activation may be involved in cell scattering.

The paper of Browne et al (J Immunol. 2006 Dec 15;177(12):8612-9) relates to two 8-base sequences in a 39-nucleotide region in the 3'-transcribed region of the HLA-A gene that are required for the posttranscriptional response to IFN-gamma. Stimulation of HLA-A expression by IFN-gamma requires nuclear export of HLA-A mRNA by chromosome maintenance region 1 (CRM-1). Treatment of cells with leptomycin B, a specific inhibitor of CRM-1, completely inhibited IFN-gamma induction of HLA-A. Expression of a truncated, dominant-negative form of the nucleoporin NUP214/CAN, DeltaCAN, that specifically interacts with CRM-1, also prevented IFN-gamma stimulation of HLA-A, providing confirmation of the role of CRM-1. Increased expression of HLA-A induced by IFN-gamma also requires protein methylation, as shown by the fact that treatment of SK-N-MC cells or HeLa cells with the PRMT1 inhibitor 5'-methyl-5'-thioadenosine abolished the cellular response to IFN-gamma. In contrast with HLA-A, IFN-gamma-induced expression of the HLA class Ib gene, HLA-E, was not affected by either 5'-methyl-5'-thioadenosine or leptomycin B. These results provide proof of principle that it is possible to differentially modulate the IFN-gamma-induced expression of the HLA-E and HLA-A genes, whose products often mediate opposing effects on cellular immunity to tumor cells, pathogens, and autoantigens.

In particular, tumor cells express FGF2. Thus, inhibitors of FGF2 secretion and/or signaling seem to be potential targets for tumor therapy. Thus, there is a need for an assay allowing for instance high-throughput screens of compound libraries for effective inhibitors of FGF2. There is a need for an assay being capable of measuring a physiological response to the presence of FGF2.

### SUMMARY OF THE INVENTION

The scope of protection is defined by the appended claims. The present disclosure provides a method for the identification of FGF2 modulating compounds, the method comprising the use of target cells sensitive to the presence of FGF2 by obstructing proliferation, wherein the target cells constitutively express a first reporter in the nucleus of living target cells, and wherein the target cells may be co-incubated with cells or a liquid to detct the sectretion or presence of FGF2 or compounds effecting FGF2. The first reporter can be a first fluorescent protein.

A second reporter may further be used for detecting apoptotic cells and the second reporter may be a second fluorescent protein.

The absorption spectrum of the first and second fluorescent protein may differ so that they can even be distinguished using a microscope.

It is intended that the fluorescence of first and/or second reporter will be determined and may form the basis for calculating the quotient of the fluorescence of the first and second reporter.

The target cells may further be co-incubated with FGF2 secreting cells and the FGF2 secrection in the FGF2 secreting cells may be inducible.

The FGF2 secreting cells can be coincubated with at least one compound selected from the group comprising peptides, proteins, nucleic acids, carbohydrates, antibodies, lipids, micelles, vesicles, synthetic molecules and polymers.

The number of living target cells may be monitored by determining fluorescence of the first reporter.

The FGF2 secreting cells may be selected from the group comprising tumor cells, endothelial cells, muscle cells, neural cells and fibroblasts.

It is further intended that additionally control cells may be used, which are constitutively expressing a third reporter, wherein the control cells are insensitive to the presence of FGF2.

The target cells may be genetically modified cells derived from a neuroblastoma, wherein the target cells may be SK-N-MC cells.

It is further envisaged that at least one compound library can be used for co-incubation with the target cells, with compounds being bound to at least one of metal particles, nanoparticles, or a solid phase and the compounds being selected from the group comprising peptides, proteins, carbohydrates, antibodies, lipids, micelles, vesicles, synthetic or biological molecules and polymers.

The method may further employ at least one compound library that may be used for co-incubation with the target cells, wherein the compounds of the library are in solution.

The target cells may be cultivated in multiwell plates with each well comprising different compounds to be tested for their ability to modulate FGF2 secretion and/or signalling.

The FGF2 secreting cells may be separated from target cells by cell sorting, such as fluorescence activated cell sorting or magnetic cell sorting, and re-cultured for further approaches.

### BRIEF DESCRIPTION OF THE FIGURES

- Figure 1: Proliferation of target cells expressing constitutively a fluorescent protein in the presence and absence of FGF2.
- Figure 2: Proliferation of target cells expressing constitutively a fluorescent reporter co-incubated with HeLa cells secreting FGF2 inducible with doxycycline.
- Figure 3/4: Effect of different FGF2 concentrations on SK-N-MC cell proliferation
- Figure 5/6: Effect of different secreted FGF2 concnetrations on SK-N-MC cells co-incubated with HeLa cells secreting FGF2 inducible with doxycycline.
- Figure 7: Quotient of red fluorescent nuclei and green fluorescence caused by apoptotic cells in the absence and presence of FGF2.
- Figure 8: Quotient of red fluorescent nuclei and green fluorescence caused by apoptotic cells in the absence and presence of doxycycline induced FGF2 secretion.
- Figure 9: Effect of different FGF2 concnetrations on SK-N-MC cells on the quotitient of red fluorescent nuclei and green fluorescence caused by apoptotic cells.
- Figure 10: Effect of secreted FGF2 concentrations on SK-N-MC cells co-incubated with HeLa cells secreting FGF2 inducible by doxycycline on the quotient of red fluorescent nuclei and green fluorescence caused by apoptotic cells.

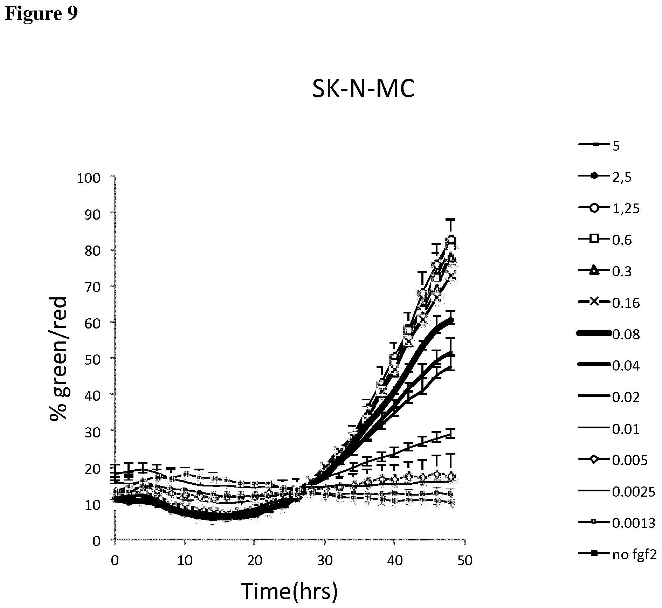

- Figure 9: Titration of recombinant FGF2 and ist effect on target cells

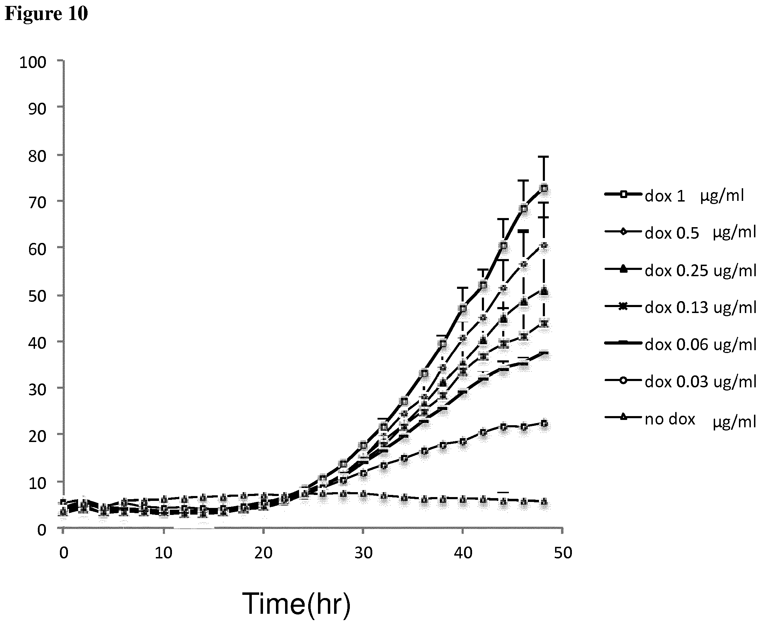

- Figure 10: Dependence of ratio of apoptotic cells to living cells from induced FGF2 concentration secreted by coincubated HeLa cells

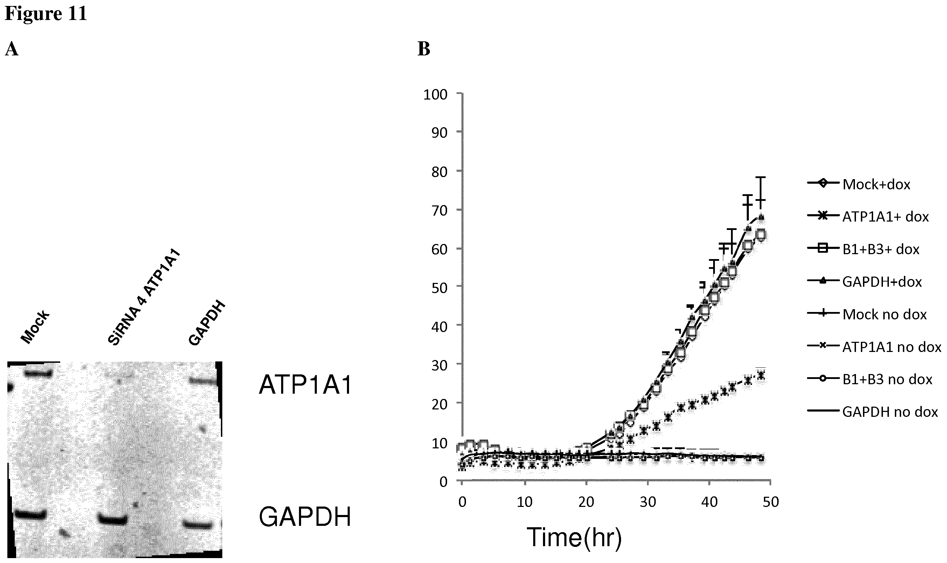

- Figure 11: Dependence of quotient of apoptotic cells to living cells from knock-down of ATP1A1 and the presence of doxycycline and GAPDH: A-Expression of ATP1A1; B-Effects of knock down

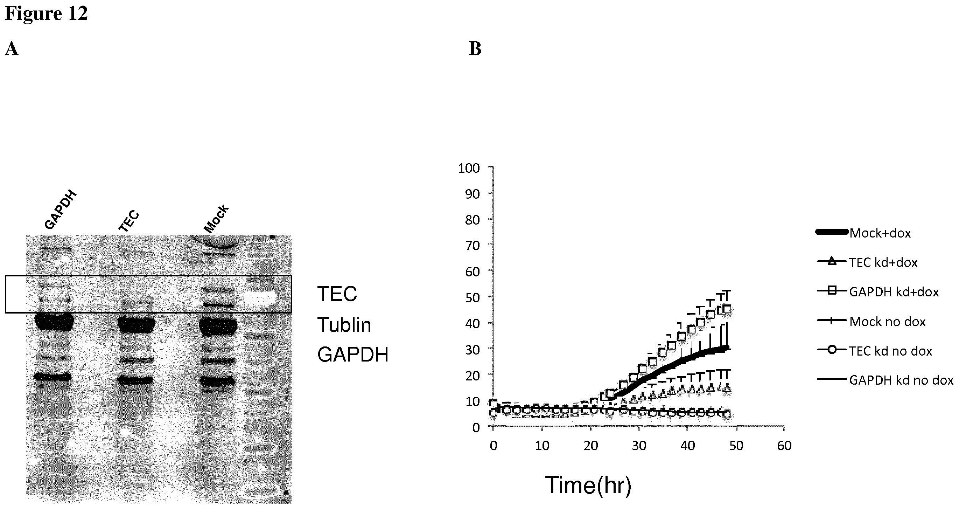

- Figure 12: Dependence of ratio of apoptotic cells to living cells from knock-down of Tec-kinase and the presence of doxycycline: A - Expression of Tec-kinase; B - Effects of knock down.

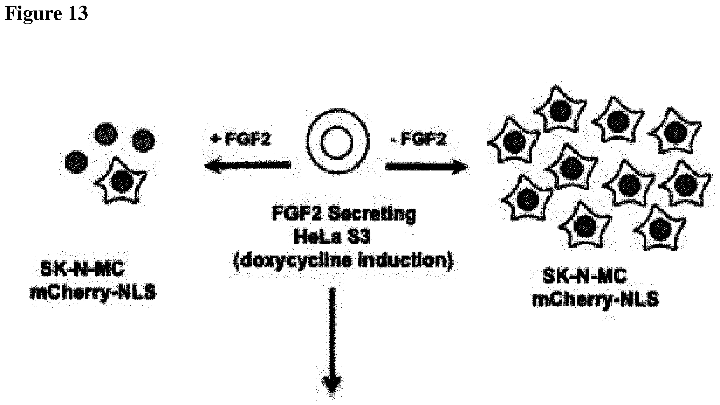

- Figure 13: Schematic depiction of quantitative assay

### DETAILLED DESCRIPTION OF THE INVENTION

The present disclosure provides a method for the identification of compounds modulating the secretion of FGF2 or FGF2 signaling by employing experiments distinguishing the absence versus presence of recombinant FGF2.

Modulation of FGF2 secretion according to the present disclosure refers to the activation or suppression of FGF2 signaling. In this context, modulation of FGF2 secretion may also refer to influencing the nature or the character of FGF2 and its secretion, either by affecting the level of FGF2 intra- or extracellular, which is still developing or maturing or by blocking secretion of FGF2 into the EM or by altering its molecular signaling capacity by blocking its binding to receptors. The disclosed assay might also reveal compounds that block downstraem signaling in target cells, like SK-N-MC cells. However, the disclosed methods provides besides quantification of FGF2 that is present a tool allowing qualitative analysis. IF target cells will be unaffected by co-incubation with other cells or compounds, the addition of recombinant FGF2 allows to determine whether the signalling pathway of the target cells is affected. If recombinant FGF2 is able to induce target cells to proliferation it becomes obvious that the before observed lack of proliferation oft the target cells is not related to the signalling pathways in the target cells. In case that the addition of recombinant FGF2 will not be able to induce proliferation of the target cells, the compound or co-incubated cells will affect the cellular machinery of the target cells or the recombinant FGF2 directly.

According to the present disclosure, target cells represent a cell line, which is sensitive to the presence of FGF2, caused by a shut-down of cell proliferation and subsequent cell death. FGF2 secreting cells represent a cell line, which is able to secret FGF2 and control cells represent a cell line, which is insensitive to the presence of FGF2.

A reporter within the meaning of the present disclosure shall be understood as a gene encoding a protein, which can be used as a reporter like a fluorescent protein. The target cells may have been stably transfected with such a gene of a first reporter, so that it will be constitutively expressed. The control cells may have been stably transfected with such a gene of a different second reporter, so that it will be constitutively expressed.

A compound library according to the present disclosure refers to a collection of chemicals, further called compounds, and their related information. Compounds are selected from the group comprising peptides, proteins, carbohydrates, antibodies, lipids, micelles, vesicles, synthetic molecules and polymers.

A "solid phase" to which compounds to be investigated can be covalently or non-covalently attached refers to, but is not restricted to, a column, a matrix, beads, glass including modified or functionalized glass, synthetic membranes, silica or silica-based materials including silicon and modified silicon, plastics (comprising polypropylene, polyethylene, polystyrene and copolymers of styrene and other materials, acrylics, polybutylene, polyurethanes etc.), nylon or nitrocellulose, resins, polysaccharides, carbon as well as inorganic glasses, metals, nanoparticles, and plastics. Thus, microtiter plates are also within the scope of a solid phase according to the present disclosure.

The method according to the disclosure may be used very efficiently for the identification of cell growth disorders, wherein cell growth disorders comprise tumour or cancer associated diseases like leukaemia. The tumour disease can be a disease selected from the group comprising tumours of the ear-nose-throat region, comprising tumors of the inner nose, nasal sinus, nasopharynx, lips, oral cavity, oropharynx, larynx, hypopharynx, ear, salivary glands, and paragangliomas, tumors of the lungs comprising non-parvicellular bronchial carcinomas, parvicel- lular bronchial carcinomas, tumors of the mediastinum, tumors of the gastrointestinal tract, comprising tumors of the esophagus, stomach, pancreas, liver, gallbladder and biliary tract, small intestine, colon and rectal carcinomas and anal carcinomas, urogenital tumors comprising tumors of the kidneys, ureter, bladder, prostate gland, urethra, penis and testicles, gynecological tumors comprising tumors of the cervix, vagina, vulva, uterine cancer, malignant trophoblast disease, ovarial carcinoma, tumors of the uterine tube (Tuba Faloppii), tumors of the abdominal cavity, mammary carcinomas, tumors of the endo- crine organs, comprising tumors of the thyroid, parathyroid, adrenal cortex, endocrine pancreas tumors, carcinoid tumors and carcinoid syndrome, multiple endocrine neoplasias, bone and soft-tissue sarcomas, mesotheliomas, skin tumors, melanomas comprising cutaneous and intraocu- lar melanomas, tumors of the central nervous system, tumors during infancy, comprising retinoblastoma, Wilms tumor, neurofibromatosis, neuroblastoma, Ewing sarcoma tumor family, rhabdomyosarcoma, lymphomas comprising non- Hodgkin lymphomas, cutaneous T cell lymphomas, primary lymphomas of the central nervous system, morbus Hodgkin, leukemias comprising acute leukemias, chronic myeloid and lymphatic leukemias, plasma cell neoplasms, myelodysplasia syndromes, paraneoplastic syndromes, metastases with unknown primary tumor (CUP syndrome) , peritoneal carcinomatosis, immunosuppression-related malignancy comprising AIDS-related malignancy such as Kaposi sarcoma, AIDS- associated lymphomas, AIDS-associated lymphomas of the central nervous system, AIDS-associated morbus Hodgkin and AIDS-associated anogenital tumors, transplantation- related malignancy, metastasized tumors comprising brain metastases, lung metastases, liver metastases, bone me- tastases, pleural and pericardial metastases, and malignant ascites.

The invention will be described by experiments and figures.

SK-N-MC cells, a human neuroepithilioma cell line (ATCC HTB-10), were stably transduced with a construct carrying the red fluorescent protein mCherry fused to a nuclear localization signal (mCherry-NLS). The cell line used in the shown experiments is derived from a single clone that was isolated by FACS resulting in homogenous red fluorescence in the nuclei of this cell population. Using an Essen Biosciences Incucyte Zoom LED microscope, the proliferation of these cells can be monitored in a quantitative manner for an almost unlimited period of time.

Since SK-N-MC cells are known to respond to FGF2 by a shut-down of cell proliferation, they can be used to sense FGF2 in the extracellular space. For example, as a proof of principle, this property of FGF2 can be demonstrated by challenging SK-N-MC cells with recombinant FGF2 (25 ng/ml). Furthermore, we can make use of this phenomenon to monitor FGF2 secretion from a second cell line based on co-cultivation of SK-N-MC cells with for example Hela S3 cells (ATCC CCL-2.2) expressing FGF2 in a doxycycline-dependent manner.

For experiments using recombinant FGF2, SK-N-MC cells expressing mCherry-NLS were cultivated in 96 well plates (Corning) at a starting density of 5000 cells per well. Cells were grown in Minimum Essential Medium Eagle alpha modified (α-MEM, Sigma) supplemented with 10% FCS, Penicillin (100 U/ml) and Streptomycin (100 µg/ml). Recombinant FGF2 (18 kDa form with N-terminal His-tag) was added at a final concentration of 25 ng/ml in α-MEM.

For co-culturing experiments, HeLa S3 cells expressing FGF2 were seeded at 1000 cells per well in 96 well plates (Corning) followed by the addition of doxycycline (1 µg/ml) to induce FGF2 expression and secretion. Following 48 hours of cultivation, SK-N-MC cells constitutively expressing mCherry-NLS were added at a density of 5000 cells per well. Cells were grown in α-MEM in an appropriate incubator at 5% CO₂ and 37°C.

To specifically monitor cell proliferation of SK-N-MC cells labelled with mCherry-NLS, live imaging was performed using an Essen Biosciences Incucyte Zoom Instrument along with a statistical analysis of three technical replicates for each experimental condition. Images were acquired every 2 hours with four images taken per well for each time point. This system allows for an absolute quantification of fluorescent nuclei and, therefore, a direct measurement of cell proliferation over time.

Since SK-N-MC cells will become apoptatic in the presence of FGF2, a caspase 3/7 assay was used to detect DNA of apoptotic cell with a green fluorescent dye. Therorectically, FGF2 should result in a decrease of red nuclei of living cells and an increase of green colour caused by an increasing amount of apoptotic cells among red SK-N-MC cells.

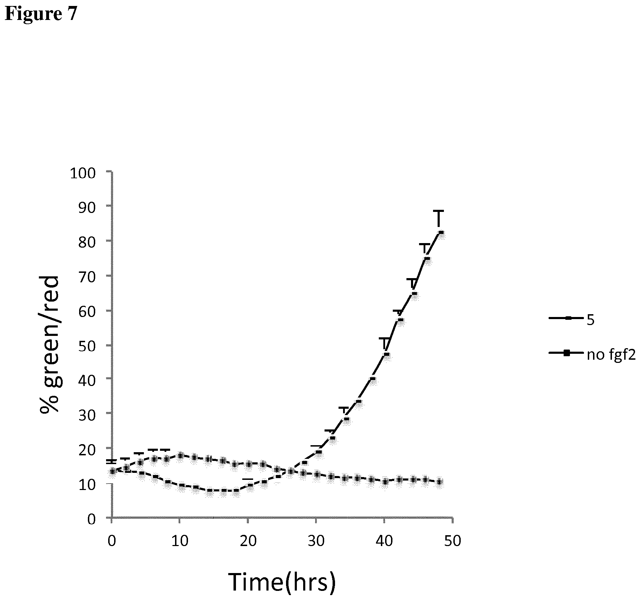

By calculating the ratio of green versus red fluorescence it should be possible to get a more precise result or relation between FGF2 concentration change in the proliferation of the target cells. Corresponding results are shown in figures 7 to 10. FGF2 was titrated and added directly or FGF2 secretion was induced by adding doxycycline to HeLa cells comprising an doxycycline inducible FGF2 gene.

Surprisingly the calculation of the quotient of red fluorescent nuclei and green fluorescence caused by apoptotic cells resulted in more robust results reflecting the effect of the respective concentration of FGF2 on the proliferation of the target cells.

A key approach in the identification of molecular components involved in FGF2 secretion has been a genome-wide RNAi screen that led to the identification of Tec kinase as a regulatory component of FGF2 secretion (Nickel et al., 2011, Traffic 12, 799-805,32; Ebert, et al., 2010), Traffic 11, 813-82). In addition to Tec kinase, this screen also revealed ATP1A1 as a gene product whose down-regulation causes a substantial drop in FGF2 secretion efficiency.

Knock-downs of ATP1A1 and Tec-kinase were done in the FGF2 expressing cell line followed by quantifying FGF2 secretion using the discosed assay employing SK-N-MC cells.

The instant disclosure discloses an assay employing a target cell line sensitive to FGF2 secretion that can be used to quantify the concentration of FGF2 and allows to determine whether inhibition of FGF2 secretion is caused in the FGF2 donor cells or the target cell line. Thus, it is not only possible to identify FGF2 secretion and/or signalling effecting molecules, but also to get information about their mode of action. Using the method utilising one or two flusorescent parameters allows for a precise determination of presence and - if a calibration took place prior to measuring for FGF2 - concentration of FGF2.

### DETAILLED DESCRIPTION OF THE FIGURES

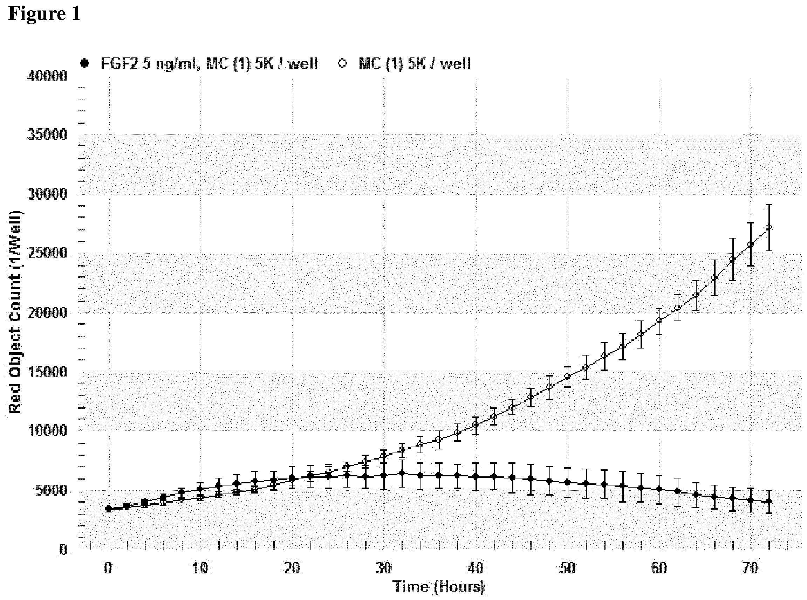

Figure 1 shows a target cell line, which is sensitive to the presence of FGF2. The cells express constitutively a red fluorescent protein coupled to a nuclear localisation signal, so that the nuclei of living cells appear to be red. The presence of recombinant FGF2 (5 ng/ml) results in a reduction of cells. The target cells are neuroblastoma cells (Neuroblastoma SK-N-MC) that were stably transfected with a red fluorescent protein. In the absence of recombinant FGF2 the cells proliferate normally.

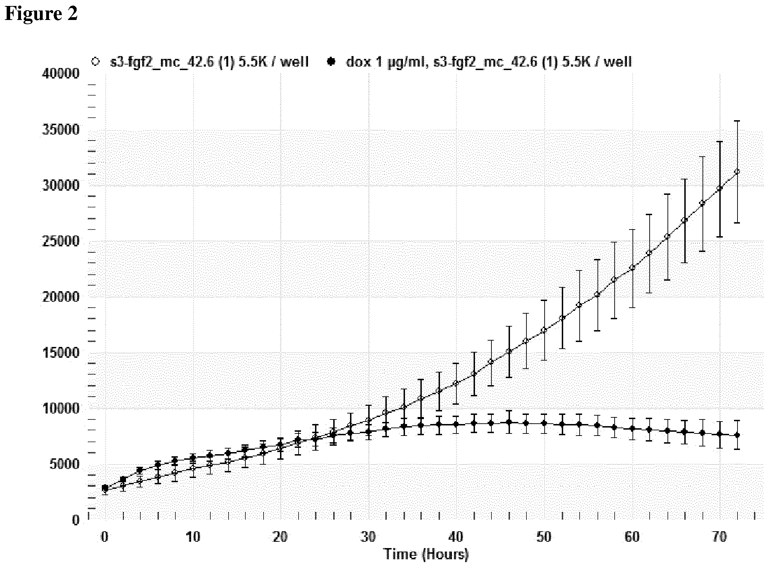

Figure 2 shows the results of co-incubating target cells expressing constitutively a RED fluorescent reporter with HeLa cells secreting FGF2 inducible by doxycycline. The upper line in figure 1 shows co-incubation of the cells without inducing FGF2 secretion by doxycycline. After induction of FGF2 secretion by addition of doxycycline a massive reduction of the number of target cells can be observed.

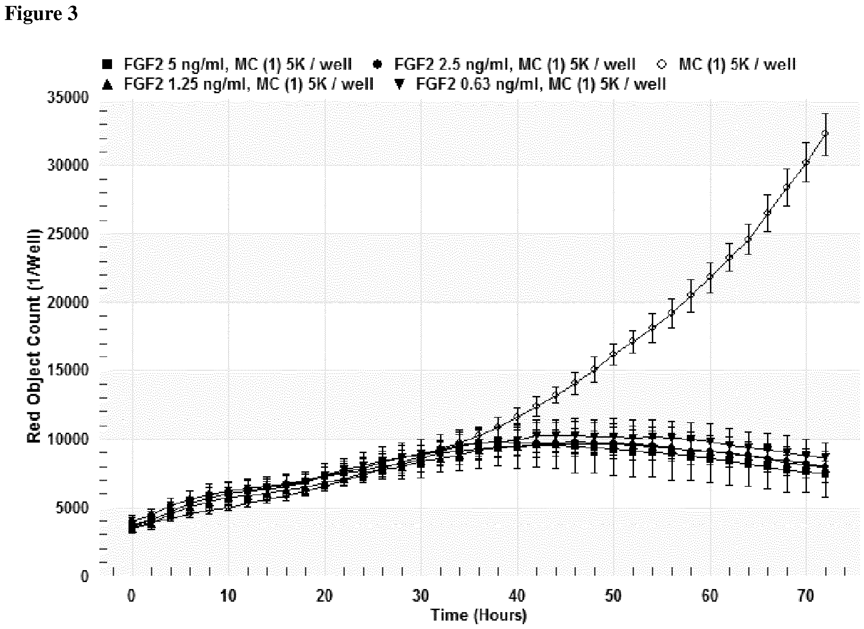

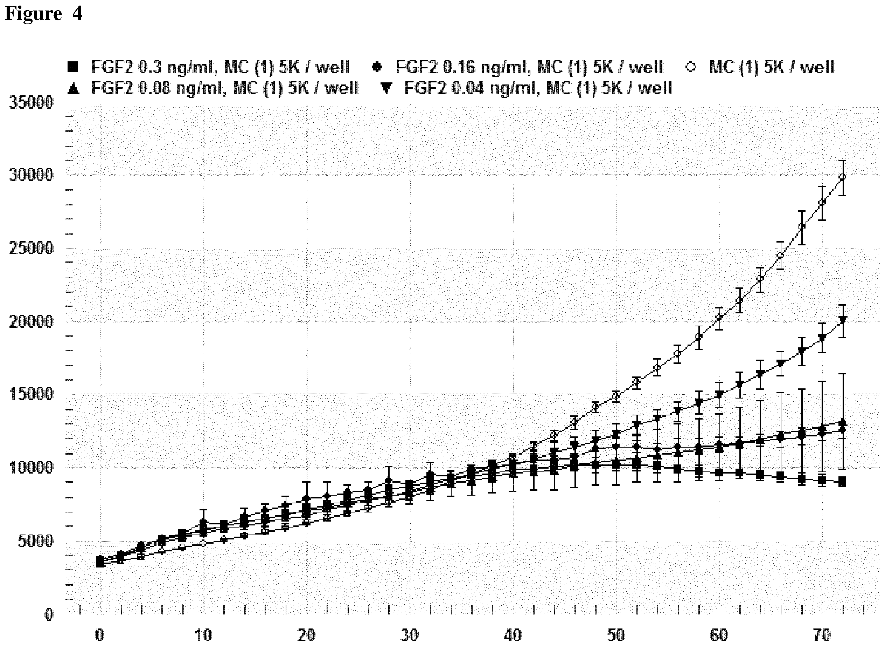

Figure 3 and 4 show effects of different concentrations of FGF2 on the cell growth of the target cell line sensitive to the presence of FGF2. It can be observed that the growth curves depend on the respective concentration of FGF2, so that less FGF2 results in a better growth of the cells.

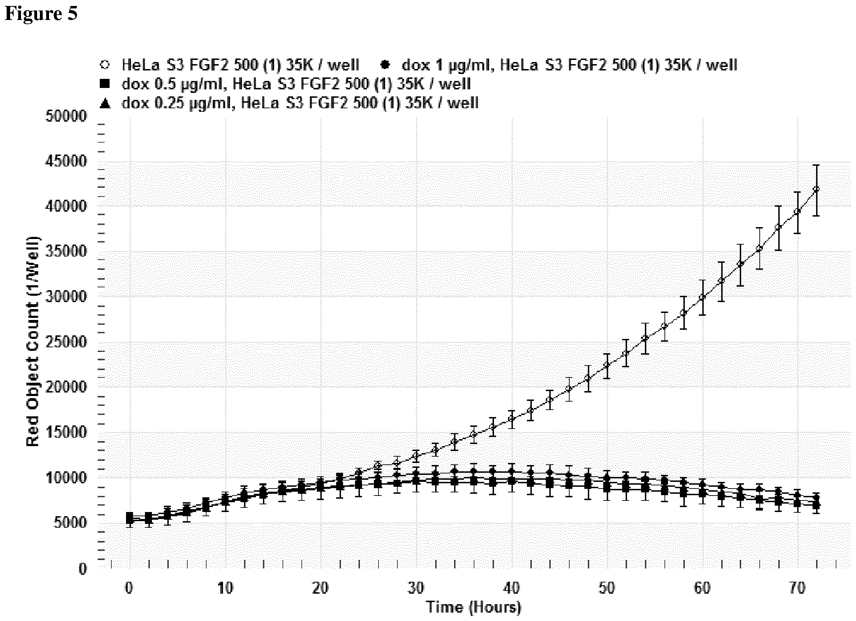

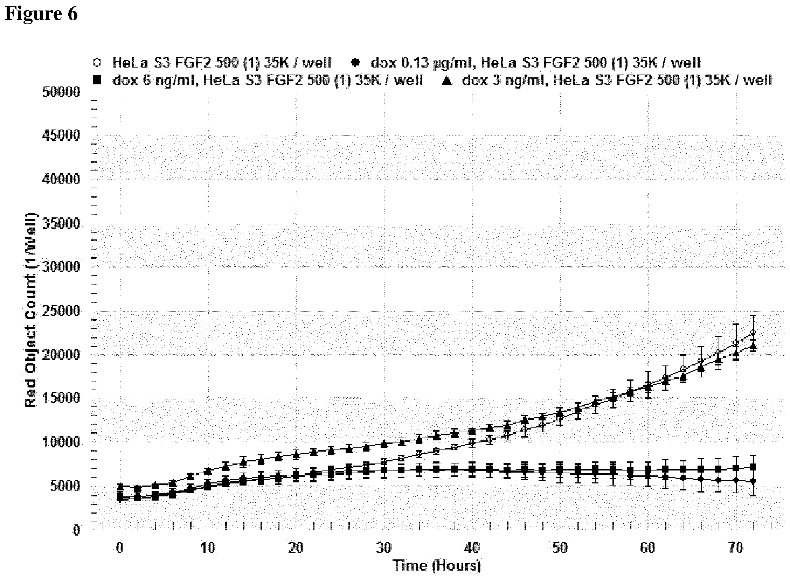

Figures 5 and 6 show effects of adding different amounts of doxycycline to the medium in order to induce FGF2 secretion by co-incubated HeLa cells. Again, the amount of induced FGF2 secretion by the addition of doxycycline (dox) effect the proliferation of the target cells in a way that the more FGF2 is secreted the less proliferation of target cells can be observed.

The results in figures 3-6 show that the proliferation of the target cells is dose dependent with respect to the presence of FGF2 even if FGF2 secretion is the effect of adding different amounts of on inducer for FGF2 secretion. Thus, the assay seems to be surprisingly suitable to prepare calibrated growth curves so that a quantification of FGF2 concentrations will be possible.

Figure 7 shows the quotient of red fluorescent nuclei and green fluorescence caused by apoptotic cells in the absence and presence of FGF2. It has to be noted that the output of the results reverses the previously shown curves, because the induction of apoptosis by FGF2 will result in an increase of the quotient of green to red fluorescence.

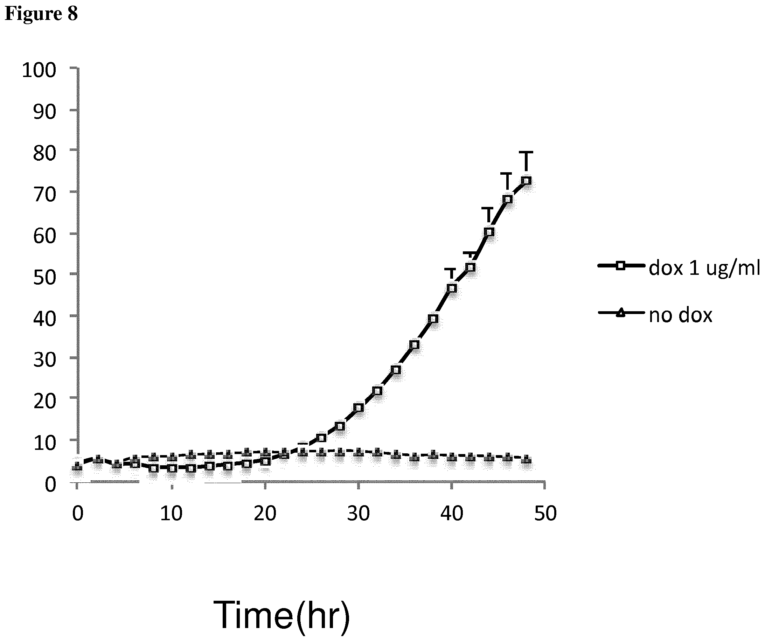

Figure 8 shows the quotient of red fluorescent nuclei and green fluorescence caused by apoptotic cells in the absence and presence of doxycycline induced FGF2 secretion. The quotient increases if doxycycline is added in a concentration of 1 µg/ml due to the increase of apoptotic cells.

Figure 9 shows the effect of different FGF2 concnetrations on SK-N-MC cells on the quotient of red fluorescent nuclei and green fluorescence caused by apoptotic cells. The quotient is dose dependent and reflects the dose dependency more precisely than using only red fluorescence of nuclei of living target cells.

Figure 10 shows the effect of secreted FGF2 concentrations on SK-N-MC cells co-incubated with HeLa cells secreting FGF2 inducible by doxycycline on the quotient of red fluorescent nuclei and green fluorescence caused by apoptotic cells. Different doxycycline concentrations were added which is reflected in the quotient of living and apoptotic cells.

Figure 11 shows in part A the knock down of ATP1A1 expression ond in part B the effect of the knock down and GAPDH (control knock-down) and/or doxycycline addition. It was surprisingly possible to detect the knock-down of ATP1A1 by using the assay calculating the quotient of red and green fluorescence caused by living cells (red) and apoptotic cells (green).

Figure 12 shows in part A the knock down of Tec-kinaseexpression ond in part B the effect of the knock down and GAPDH (control knock-down) and/or doxycycline addition. It was surprisingly possible to detect the knock-down of Tec-kinase as well by using the assay calculating the quotient of red and green fluorescence caused by living cells (red) and apoptotic cells (green).

## Claims

1. A method for the identification of the presence of FGF2, the method comprising the use of target cells sensitive to the presence of FGF2 by obstructing proliferation, wherein the target cells constitutively express a first reporter in the nucleus of living target cells, and wherein the target cells are co-incubated with cells or a liquid to detect the secretion or presence of FGF2.

2. The method of claim 1, wherein the first reporter is a first fluorescent protein.

3. The method of claim 1 or 2, wherein a second reporter is used for detecting apoptotic cells.

4. The method of claim 3, wherein the second reporter is a second fluorescent protein.

5. The method of claim 4, wherein the absorption spectrum of the first and second fluorescent protein differ.

6. The method according to any one of claims 1 to 5, wherein the fluorescence of first and/or second reporter is determined.

7. The method of claim 6, wherein the quotient of the fluorescence of the first and second reporter is calculated.

8. The method of claim 1 to 7, wherein the target cells are co-incubated with FGF2 secreting cells

9. The method of claim 8, wherein the FGF2 secrection in the FGF2 secreting cells is inducible.

10. The method of any one of claims 8 or 9, wherein the FGF2 secreting cells are coincubated with at least one compound selected from the group comprising peptides, proteins, nucleic acids, carbohydrates, antibodies, lipids, micelles, vesicles, synthetic molecules and polymers.

11. The method of any one of claims 1 to 10, wherein the number of living target cells monitored by determining fluorescence of the first reporter.

12. The method of any one of claims 8 to 11, wherein the FGF2 secreting cells are selected from the group comprising tumor cells, endothelial cells, muscle cells, neural cells and fibroblasts.

13. The method of any one of claims 1 to 12, wherein additionally control cells are used, which are constitutively expressing a third reporter, wherein the control cells are insensitive to the presence of FGF2.

14. The method of one of claims 1 to 13, wherein the target cells are genetically modified cells derived from a neuroblastoma.

15. The method of any one of claims 1 to 14, wherein the target cells are SK-N-MC cells.

16. The method of one of claims 1 to 15, wherein at least one compound library is used for co-incubation with the target cells, with compounds being bound to at least one of metal particles, nanoparticles, or a solid phase and the compounds being selected from the group comprising peptides, proteins, carbohydrates, antibodies, lipids, micelles, vesicles, synthetic or biological molecules and polymers.

17. The method of one of claims 1 to 16, wherein at least one compound library is used for co-incubation with the target cells, wherein the compounds of the library are in solution.

18. The method of one of claims 1 to 17, wherein the target cells are cultivated in multiwell plates with each well comprising different compounds to be tested for their ability to modulate FGF2 secretion and/or signalling.

19. The method of one of claims 1 to 18, wherein FGF2 secreting cells are separated from target cells by cell sorting, such as fluorescence activated cell sorting or magnetic cell sorting, and re-cultured for further approaches.

## Patentansprüche

1. Ein Verfahren zum Nachweis des Vorhandenseins von FGF2, wobei das Verfahren die Verwendung von Zielzellen umfasst, die für das Vorhandensein von FGF2 empfindlich sind, indem sie die Proliferation behindern, wobei die Zielzellen einen ersten Reporter im Kern lebender Zielzellen konstitutiv exprimieren, und wobei die Zielzellen mit Zellen oder einer Flüssigkeit ko-inkubiert werden, um die Sekretion oder das Vorhandensein von FGF2 nachzuweisen.

2. Das Verfahren nach Anspruch 1, wobei der erste Reporter ein erstes fluoreszierendes Protein ist.

3. Das Verfahren nach Anspruch 1 oder 2, wobei ein zweiter Reporter zum Nachweis apoptotischer Zellen verwendet wird.

4. Das Verfahren nach Anspruch 3, wobei der zweite Reporter ein zweites fluoreszierendes Protein ist.

5. Das Verfahren nach Anspruch 4, wobei sich das Absorptionsspektrum des ersten und zweiten fluoreszierenden Proteins unterscheidet.

6. Das Verfahren nach einem der Ansprüche 1 bis 5, wobei die Fluoreszenz des ersten und/oder zweiten Reporters bestimmt wird.

7. Das Verfahren nach Anspruch 6, wobei der Quotient aus der Fluoreszenz des ersten und zweiten Reporters berechnet wird.

8. Das Verfahren nach Anspruch 1 bis 7, wobei die Zielzellen mit FGF2-sezernierenden Zellen ko-inkubiert werden.

9. Das Verfahren nach Anspruch 8, wobei die FGF2-Sekretion in den FGF2-sekretierenden Zellen induzierbar ist.

10. Das Verfahren nach einem der Ansprüche 8 oder 9, wobei die FGF2-sezernierenden Zellen mit mindestens einer Verbindung aus der Gruppe der Peptide, Proteine, Nukleinsäuren, Kohlenhydrate, Antikörper, Lipide, Mizellen, Vesikel, synthetischen Moleküle und Polymere koubiert werden.

11. Das Verfahren nach einem der Ansprüche 1 bis 10, wobei die Anzahl der lebenden Zielzellen durch Bestimmung der Fluoreszenz des ersten Reporters überwacht wird.

12. Das Verfahren nach einem der Ansprüche 8 bis 11, wobei die FGF2-sezernierenden Zellen aus der Gruppe ausgewählt sind, die Tumorzellen, Endothelzellen, Muskelzellen, neurale Zellen und Fibroblasten umfasst.

13. Das Verfahren nach einem der Ansprüche 1 bis 12, wobei zusätzlich Kontrollzellen verwendet werden, die konstitutiv einen dritten Reporter exprimieren, wobei die Kontrollzellen unempfindlich gegenüber der Anwesenheit von FGF2 sind.

14. Das Verfahren nach einem der Ansprüche 1 bis 13, wobei die Zielzellen genetisch veränderte Zellen sind, die von einem Neuroblastom stammen.

15. Das Verfahren nach einem der Ansprüche 1 bis 14, wobei die Zielzellen SK-N-MC-Zellen sind.

16. Das Verfahren nach einem der Ansprüche 1 bis 15, bei dem mindestens eine Verbindungsbibliothek zur Co-Inkubation mit den Zielzellen verwendet wird, wobei die Verbindungen an mindestens eines der folgenden Elemente gebunden sind: Metallpartikel, Nanopartikel oder eine feste Phase, und die Verbindungen aus der Gruppe ausgewählt sind, die Peptide, Proteine, Kohlenhydrate, Antikörper, Lipide, Mizellen, Vesikel, synthetische oder biologische Moleküle und Polymere umfasst.

17. Das Verfahren nach einem der Ansprüche 1 bis 16, wobei mindestens eine Verbindungsbibliothek für die Co-Inkubation mit den Zielzellen verwendet wird, wobei die Verbindungen der Bibliothek in Lösung vorliegen.

18. Das Verfahren nach einem der Ansprüche 1 bis 17, wobei die Zielzellen in Multiwell-Platten kultiviert werden, wobei jede Vertiefung verschiedene Verbindungen enthält, die auf ihre Fähigkeit, die FGF2-Sekretion und/oder -Signalisierung zu modulieren, getestet werden.

19. Das Verfahren nach einem der Ansprüche 1 bis 18, wobei FGF2 sezernierende Zellen durch Zellsortierung, wie fluoreszenzaktivierte Zellsortierung oder magnetische Zellsortierung, von Zielzellen getrennt und für weitere Ansätze rekultiviert werden.

## Revendications

1. Une méthode d'identification de la présence de FGF2, comprenant l'utilisation de cellules cibles sensibles à la présence de FGF2 par obstruction de la prolifération, dans lesquelles les cellules cibles expriment de manière constitutive un premier rapporteur dans le noyau des cellules cibles vivantes, et dans lesquelles les cellules cibles sont co-incubées avec des cellules ou un liquide pour détecter la sécrétion ou la présence de FGF2.

2. La méthode de la revendication 1, dans laquelle le premier rapporteur est une première protéine fluorescente.

3. La méthode de la revendication 1 ou 2, dans laquelle un second rapporteur est utilisé pour détecter les cellules apoptotiques.

4. La méthode de la revendication 3, dans laquelle le second rapporteur est une seconde protéine fluorescente.

5. La méthode de la revendication 4, dans laquelle le spectre d'absorption de la première et de la seconde protéine fluorescente diffère.

6. La méthode selon l'une des revendications 1 à 5, dans laquelle la fluorescence du premier et/ou du deuxième rapporteur est déterminée.

7. La méthode de la revendication 6, dans laquelle le quotient de la fluorescence du premier et du second rapporteur est calculé.

8. La méthode des revendications 1 à 7, dans laquelle les cellules cibles sont co-incubées avec des cellules sécrétrices de FGF2.

9. La méthode de la revendication 8, dans laquelle la sécrétion de FGF2 dans les cellules sécrétrices de FGF2 est inductible.

10. La méthode de l'une des revendications 8 ou 9, dans laquelle les cellules sécrétrices de FGF2 coïncident avec au moins un composé choisi dans le groupe comprenant les peptides, les protéines, les acides nucléiques, les hydrates de carbone, les anticorps, les lipides, les micelles, les vésicules, les molécules synthétiques et les polymères.

11. La méthode de l'une des revendications 1 à 10, dans laquelle le nombre de cellules cibles vivantes est contrôlé par la détermination de la fluorescence du premier rapporteur.

12. La méthode de l'une des revendications 8 à 11, dans laquelle les cellules sécrétrices de FGF2 sont choisies dans le groupe comprenant les cellules tumorales, les cellules endothéliales, les cellules musculaires, les cellules neurales et les fibroblastes.

13. La méthode de l'une des revendications 1 à 12, dans laquelle on utilise en outre des cellules de contrôle exprimant de manière constitutive un troisième rapporteur, les cellules de contrôle étant insensibles à la présence de FGF2.

14. La méthode de l'une des revendications 1 à 13, dans laquelle les cellules cibles sont des cellules génétiquement modifiées dérivées d'un neuroblastome.

15. La méthode de l'une des revendications 1 à 14, dans laquelle les cellules cibles sont des cellules SK-N-MC.

16. La méthode de l'une des revendications 1 à 15, dans laquelle au moins une bibliothèque de composés est utilisée pour la coincubation avec les cellules cibles, les composés étant liés à au moins une des particules métalliques, des nanoparticules ou une phase solide et les composés étant choisis dans le groupe comprenant les peptides, les protéines, les hydrates de carbone, les anticorps, les lipides, les micelles, les vésicules, les molécules synthétiques ou biologiques et les polymères.

17. La méthode de l'une des revendications 1 à 16, dans laquelle au moins une bibliothèque de composés est utilisée pour la coincubation avec les cellules cibles, les composés de la bibliothèque étant en solution.

18. La méthode de l'une des revendications 1 à 17, dans laquelle les cellules cibles sont cultivées dans des plaques multipuits, chaque puits contenant différents composés à tester pour leur capacité à moduler la sécrétion et/ou la signalisation du FGF2.

19. La méthode de l'une des revendications 1 à 18, dans laquelle les cellules sécrétrices de FGF2 sont séparées des cellules cibles par tri cellulaire, tel que le tri cellulaire activé par fluorescence ou le tri cellulaire magnétique, et recultivées pour d'autres approches.
